# EUROPÄISCHE PATENTSCHRIFT

(11) **EP 1 434 781 B1**
(45) Veröffentlichungstag und Bekanntmachung des Hinweises auf die Patenterteilung: **26.07.2006**
(21) Anmeldenummer: 02800574.2
(22) Anmeldetag: 26.09.2002
(51) Int. Cl.: C07F 9/6568, C07F 9/6571, B01J 31/24, C07C 67/38

(54) **DIPHOSPHIN**
DIPHOSPHINE
DIPHOSPHINE

(30) Priorität: 02.10.2001 DE 10148712
(43) Veröffentlichungstag der Anmeldung: 07.07.2004
(73) Patentinhaber: BASF Aktiengesellschaft, 67056 Ludwigshafen (DE)
(72) Erfinder: SAVA, Xavier, 68161 Mannheim (DE); SLANY, Michael, 67281 Kirchheim (DE); RÖPER, Michael, 67157 Wachenheim (DE)
(86) Internationale Anmeldenummer: PCT/EP2002/010798
(87) Internationale Veröffentlichungsnummer: WO 2003/031457

(56) Entgegenhaltungen:
- EP-A- 0 273 489
- WO-A-01/87899
- WO-A-98/42717
- GEE V ET AL: "BIS(PHOSPHA-ADAMANTYL)ALKANES: A NEW CLASS OF VERY BULKY DIPHOSPHINES" CHEMICAL COMMUNICATIONS, ROYAL SOCIETY OF CHEMISTRY, GB, Nr. 10, 1999, Seiten 901-902, XP001010653 ISSN: 1359-7345
- PUGH R.I. ET AL: "Tandem isomerisation-carbonalation catalysis: highly active palladium(II) catalysts for the selective methoxycarbonylation of internal alkenes to linear esters" CHEMICAL COMMUNICATIONS., Nr. 16, - 21. August 2001 (2001-08-21) Seiten 1476-1477, XP002226559 ROYAL SOCIETY OF CHEMISTRY., GB ISSN: 1359-7345

## Beschreibung

Die vorliegende Erfindung betrifft ein Diphosphin der Formel

R¹>P-(CH₂)ₙ-PR²R³

wobei
R¹ ein bivalentes Radikal ist, das zusammen mit dem Phosphor-Atom, mit dem es verknüpft ist, eine unsubstituierte oder substituierte 2-phospha-tricyclo[3.3.1.1{3,7}]decyl-Gruppe oder ein Derivat hiervon darstellt, in dem eines oder mehrere der Kohlenstoffatome durch Heteroatome ersetzt sind,
R², R³ unabhängig voneinander ein einwertiges Radikal mit 1 bis 20 Atomen oder ein gemeinsames zweiwertiges Radikal mit 2 bis 20 Atomen,
n 4 oder 5 sind
und deren Gemische.

Ferner betrifft sie ein als Carbonylierungskatalysator geeignetes System, enthaltend Palladium oder eine Palladiumverbindung und ein solches Diphosphin, sowie ein Verfahren zur Carbonylierung eines konjugierten Diens in Gegenwart eines solchen Systems.

Alkencarbonsäuren und deren Derivate stellen wichtige Zwischenstufen in der organischen Synthese, beispielsweise von Wirkstoffen und Polymeren, dar. So kann beispielsweise Pentensäure oder deren Derivate, wie Ester, durch weitere Funktionalisierung zu Adipinsäurederivaten oder 6-Aminocapronsäurederivaten umgesetzt werden. Adipinsäure und 6-Aminocapronsäure stellen wichtige Ausgangsverbindungen zur Herstellung technisch bedeutsamer Polymere, insbesondere Polyamide, dar.

Verfahren zur Herstellung von Alkencarbonsäurederivaten durch Carbonylierung von konjugierten Dienen in Anwesenheit einer hydroxylgruppenhaltigen Verbindung in flüssiger Phase in Gegenwart eines als Carbonylierungskatalysator geeigneten Systems enthaltend eine Palladiumverbindung und einen multidentat-organischen Phosphorliganden sind bekannt, beispielsweise aus EP 273 489 A1 oder EP 495 547 A2.

Nachteilig bei diesen Verfahren ist, daß sich Palladium als Metall abscheidet und damit dem katalytisch aktiven System entzogen wird. Zudem kann das metallische Palladium nur in technisch aufwendiger Weise aus dem Reaktionsgemisch zurückgewonnen werden.

Aus WO 98/42717 ist bekannt, daß bei der Carbonylierung von Olefinen in Gegenwart von als Katalysator geeigneten Systemen enthaltend Palladium oder eine Palladiumverbindung und einen multidentat-organischen Phosphorliganden die Aktivität erhöht werden kann, indem man als multidentat-organischen Phosphorliganden ein Diphosphin der Formel R¹>P-R²-PR³R⁴, in der R¹ für eine unsubstituierte oder substituierte 2-phospha-tricyclo[3.3.1.1{3,7}]decyl-Gruppe, R² für ein Brückengruppe, R³, R⁴ unabhängig voneinander für ein einwertiges Radikal mit 1 bis 20 Kohlenstoffatomen oder ein gemeinsames zweiwertiges Radikal mit 2 bis 20 Kohlenstoffatomen steht, einsetzt. Konkret genannt ist als R² nur die 1,2-Ethan- und 1,3-Propan-Gruppe. Solche Diphosphine scheinen gemäß den in WO 98/42717 genannten Beispielen im Falle der Carbonylierung von Ethylen, Propen, alpha-C₁₄-Olefinen und Methyl-3-pentenoat zu hohen Aktivitäten öder Selektivitäten zu führen. Wendet man aber solche Systeme auf konjugierte Diene, wie 1,3-Butadien, an, so ist die Selektivität drastisch schlechter als in der bereits o.g. EP 273 489 A1.

Der vorliegenden Erfindung lag die Aufgabe zugrunde, ein als Carbonylierungskatalysator geeigneten Systems enthaltend eine Palladiumverbindung und einen multidentat-organischen Phosphorliganden bereitzustellen, das die Carbonylierung eines konjugierten Diens in Anwesenheit einer hydroxylgruppenhaltigen Verbindung in flüssiger Phase in guten Ausbeuten, hohen Selektivitäten und reduzierte Palladium-Äbscheidung auf technisch einfache und wirtschaftliche Weise ermöglicht.

Demgemäß wurde das eingangs definierte Diphosphin, ein als Carbonylierungskatalysator geeignetes System, enthaltend Palladium oder eine Palladiumverbindung und ein solches Diphosphin, sowie ein Verfahren zur Carbonylierung eines konjugierten Diens in Gegenwart eines solchen Systems gefunden.

Erfindungsgemäß stellt R¹ ein bivalentes Radikal, das zusammen mit dem Phosphor-Atom, mit dem es verknüpft ist, eine 2-Phospha-tricyclo[3.3.1-1{3,7}]decyl-Gruppe oder ein Derivat hiervon dar, in dem eines oder mehrere der Kohlenstoffatome durch Heteroatome ersetzt sind. Zum besseren Verständnis der vorliegenden Erfindung sei darauf verwiesen, daß Tricyclo[3.3.1.1{3,7}]decan der systematische Name für eine Verbindung ist, die allgemein als "Adamantan" bekannt ist.

In einer vorteilhaften Ausführungsform kann R¹ zusammen mit dem Phosphor-Atom, mit dem es verknüpft ist, eine unsubstituierte 2-Phospha-tricyclo[3.3.1.1{3,7}]decyl-Gruppe darstellen.

In einer vorteilhaften Ausführungsform kann R¹ zusammen mit dem Phosphor-Atom, mit dem es verknüpft ist, eine substituierte, vorzugsweise in einer oder mehreren der Position 1, 3, 5 oder 7 durch ein einwertiges Radikal R⁴ substituierte, 2-Phospha-tricyclo [3.3.1.1{3,7}]decyl-Gruppe darstellen. Als R⁴ kommt vorteilhaft ein Radikal mit 1 bis 20 Atomen in Betracht, wie Methyl, Trifluormethyl; Ethoxy, Phenyl oder 4-Dodecylphenyl in Betracht. In einer besonders bevorzugten Ausführungsform kann jede der Positionen 1, 3, 5 und 7 substituiert sein durch, vorzugsweise gleiche, Radikale R⁴.

Vorteilhaft können im Gerüst von R¹ eines oder mehrere Kohlenstoffatome durch Heteroatome ersetzt sein, insbesondere durch Sauerstoff oder Schwefel, vorzugsweise in der Position 6, 9 und 10.

In einer bevorzugten Ausführungsform kommt als R¹ zusammen mit dem Phosphor-Atom, mit dem es verknüpft ist, die 2-Phospha-1,3,5,7-tetramethyl-6, 9,10-trioxodamanthyl-Gruppe in Betracht.

Erfindungsgemäß kann jede der Gruppen R² und R³ unabhängig voneinander ein einwertiges Radikal mit 1 bis 20 Atomen darstellen, wie Alkyl, beispielsweise Methyl, Ethyl, n-Propyl, i-Propyl, n-Butyl, i-Butyl, s-Butyl, t-Butyl, Cyclohexyl, wie Aryl, beispielsweise Phenyl, o,o-di(t-butoxy)phenyl, wie Heteroaryl, beispielsweise Pyridyl, wie unsubstituierte oder substituierte Heterohydrocarbyl, beispielsweise Trimethylsilyl, oder wie Alkoxy, beispielsweise Methoxy, Ethoxy, n-Propoxy, i-Propoxy, n-Butoxy, i-Butoxy, s-Butoky, t-Butoxy.

Ebenso können R² und R³ ein gemeinsames zweiwertiges Radikal mit 2 bis 20 Atomen bilden, wie 1,5-Pentylen, 1,6-Hexylen, 1,3-Cyclooctylen, 1,4-Cyclooctylen. Bevorzugt können R² und R³ zusammen mit dem Phosphor-Atom, mit dem sie verknüpft sind, gemeinsam eine 2-Phospha-tricyclo[3.3.1.1{3,7}]decyl-Gruppe bilden mit den bereits für R¹ beschriebenen Ausführungsformen. In einer besonders bevorzugten Ausführungsform bilden R² und R³ gemeinsam eine mit R¹ identische Gruppe.

Erfindungsgemäß ist n gleich 4 oder 5.

In einer bevorzugten Ausführungsform ist n gleich 4.

In einer anderen bevorzugten Ausführungsform ist n gleich 5.

Besonders bevorzugte Diphosphine sind solche ausgewählt aus der Gruppe bestehend aus 1,4-P,P'-di(2-phospha-1,3,5,7-tetramethyl-6,9,10-trioxatricyclo[3.3.1.1{3,7}]decyl)-butan, 1,4-P,P'-di-perfluoro-(2-phospha-1,3,5,7-tetramethyl-6,9,10-trioxatricyclo[3.3.1.1{3,7}]decyl)-butan, 1,4-P,P'-di(2-phospha-1,3,5,7-tetra(trifluormethyl)-6,9,10-trioxatricyclo[3.3.1.1{3,7}]decyl)-butan, vorzugsweise 1,4-P,P'-di(2-phospha-1,3,5,7-tetramethyl-6,9,10-trioxatricyclo[3.3.1.1{3,7}]decyl)-butan.

Besonders bevorzugte Diphosphine sind solche ausgewählt aus der Gruppe bestehend aus 1,5-P,P'-di(2-phospha-1,3,5,7-tetramethyl-6,9,10-trioxatricyclo[3.3.1.1{3,7}]decyl)-pentan, 1, 5-P,P'-di-perfluoro-(2-phospha-1,3,5,7-tetramethyl-6,9,10-trioxatricyclo [3.3.1.1{3,7}]decyl)-pentan, 1,5-P,P'-di(2-phospha-1,3,5,7-tetra(trifluormethyl)-6,9,10-trioxatricyclo-[3.3.1.1{3,7}]decyl)-pentan, vorzugsweise 1,5-P,P'-di(2-phospha-1,3,5,7-tetramethyl-6,9,10-trioxatricyclo[3.3.1.1{3,7}]decyl)-pentan.

Die Herstellung kann ähnlich der in WO 98/42717, beispielsweise für 1,2-P,P'-di(2-phospha-1,3,-5,7-tetramethyl-6,9,10-trioxatricyclo[3.3.1.1{3,7}]decyl)-ethan oder 1,3-P,P'-di(2-phospha-1,3,5,7-tetramethyl-6,9,10-trioxatricyclo[3.3.1.1{3,7}]decyl)-propan, beschriebenen Verfahren erfolgen. So kann beispielsweise 1,4-P,P'-di(2-phospha-1,3,5,7-tetrainethyl-6,9,10-trioxatricyclo[3.3.1.1{3,7}]decyl) -butan durch Umsetzung von 2,4-Pentandion mit 1,4-Diphosphinobutan oder beispielsweise 1,5-P,P'-di(2-phospha-1,3,5,7-tetramethyl-6,9,10-trioxatricyclo-[3.3.1.1{3,7}]decyl)-pentan durch Umsetzung von 2,4-Pentandion mit 1,5-Diphosphinopentan erhalten werden. Asymmetrische Diphosphine können beispielsweise hergestellt werden, indem man ein 1,4-Diphosphino-butan oder 1,5-Diphosphinopentan mit einer primären und einer tertiären Phosphino-Gruppe einsetzt. Alternativ können sie hergestellt werden durch Umsetzung eines sekundären 2-Phospha-tricyclo[3.3.1.1{3,7}]decan mit einem anderen sekundären Monophosphin oder nach anderen bekannten Verfahren. Substituierte Diphosphine können beispielsweise hergestellt werden, indem man substituierte 2,4-Pentandione, wie Perfluor-2,4-pentandion oder 1,1,1,5,5,5-Hexafluor-2,4-pentandion, in der Reaktion mit 1,4-Diphosphinobutan oder 1,5-Diphosphinopentan einsetzt.

Erfindungsgemäß enthält das als Carbonylierungskatalysator geeignete System
(I) Palladium oder eine Palldiumverbindung und
(II) eines der bereits beschriebenen Diphosphine.

Dabei kann als Komponente (II) ein bestimmtes erfindungsgemäßes Diphosphin oder ein Gemisch solcher Diphosphine eingesetzt werden.

Ebenso ist es möglich, ein bestimmtes erfindungsgemäßes Diphosphin mit einem anderen Diphosphin oder einem Gemisch solcher anderer Diphosphine, oder ein Gemisch der erfindungsgemäßen Diphosphine mit einem anderen Diphosphin oder einem Gemisch solcher anderer Diphosphine eingesetzt werden.

Im Sinne der vorliegenden Erfindung wird der Begriff "Diphosphin" für ein bestimmtes Diphosphin oder ein Gemisch von Diphosphinen gemeinsam verwendet.

Das erfindungsgemäße System kann als Carbonylierungskatalysator eingesetzt werden, wie sie beispielsweise in EP 273 489 A1, EP 495 547 A2 oder WO 98/42717 beschrieben sind; insbesondere in den verschiedenen Reaktionen, in denen Kohlenmonoxid an eine ungesättigte Verbindung, vorzugsweise eine olefinische Doppelbindung, addiert wird. Solche Reaktionen können schematisch dargestellt werden gemäß

R-CH=CH-R' + CO + HY → R=CH2-CH(-R')(-COY)

mit
Y H, OH, OR" , NR"'R""
R, R', R", R'", R'''' organischer Rest

Das erfindungsgemäße System kann demnach eingesetzt werden beispielsweise in Hydroformylierungs-Reaktionen, Hydrocarboxylierungs-Reaktionen, Hydroesterifizierungs-Reaktionen, Hydroamidierungs-Reaktionen. Als besonders vorteilhaft hat sich das erfindungsgemäße System in Hydrocarboxylierungs-Reaktionen und Hydroesterifizierungs-Reaktionen erwiesen.

Als ungesättigte Verbindung kommen vorteilhaft konjugierte Diene in Betracht, also organische Verbindungen, die die Struktur

>C=C(R^{x}) -C (R^{y}) =C<

mit R^{x}, R^{y} einwertiges Radikal
beeinhalten, insbesondere nicht-cyclische Verbindungen, wie 1,3-Butadien, 2-Methyl-1,3-butadien, 1,3-Pentadien, 1,3-Hexadien, 2,4-Hexadien, 2,3-Dimethyl-1,3-butadien, vorzugsweise 1,3-Butadien.

In den ungesättigten Verbindungen, vorzugsweise konjugierten Dienen können ein oder mehrere Wasserstoffatome durch andere Atome, wie Halogenatome, oder Gruppen von Atomen, wie Hydroxyl-Gruppen, Cyano-Gruppen, Methoxy- oder Ethoxy-Gruppen, Amino-Gruppen, wie Dimethylamino- oder Diethylamino Gruppen, Carboxyl-Gruppen, Ester-Gruppen substituiert sein.

In einer besonders bevorzugten Ausführungsform können durch Hydrocarboxylierung solcher konjugierte Diene Alkencarbonsäuren erhalten werden, beispielsweise aus 1,3-Butadien Pentensäure, wie cis-2-Pentensäure, trans-2-Pentensäure, cis-3-Pentensäure, trans-3-Pentensäure, 4 Pentensäüre.

In einer besonders bevorzugten Ausführungsform können durch Hydroesterifizierung solcher konjugierte Diene Alkencarbonsäureester erhalten werden, beispielsweise aus 1,3-Butadien Pentensäureester, wie cis-2-Pentensäureester, trans-2-Pentensäureester, cis-3-Pentensäureester, trans-3-Pentensäureester, 4-Pentensäureester.

In solchen Carbonylierunesreaktionen können weitere Komponenten, wie Koreaktanten oder flüssige Verdünnungsmittel eingesetzt werden. Ebenso können Anionen als Gegenion zu dem eingesetzten Palladiuinkation im Falle von Palladiumverbindungen eingesetzt werden. Beispiele hierzu umfassen Anionen, die die konjugierte Base einer Säure mit einem-pKa-Wert, gemessen in Wasser bei 18°C, kleiner als 6, vorzugsweise kleiner als 4 sind. Solche Anionen koordinieren nicht oder nur gering Palladium, d.h. daß keine oder nur eine geringe Wechselwirkung zwischen dem Anion und dem Palladium-Kation bestehen. Katalysatoren, die ein solches Anion enthalten, zeigen im allgemeinen eine gute Aktivität.

Geeignete Anionen umfassen Anionen, die sich ableiten von Brönstedt-Säuren, wie Phosphorsäure oder Schwefelsäure, vorzugsweise von Carbonsäuren, halogenierten Carbonsäuren oder Sulfonsäuren, wie Methansulfonsäure, Trifluortmethansulfonsäure oder p-Toluolsulfonsäure. Bevorzugt sind Anionen, die sich von Carbonsäuren ableiten, 2,6-Dichlorbenzoesäure, 2,6-Dimethoxybenzoesäure und 2,4,6-Trimethylbenzoesäure.

Ebenso können komplexe Anionen eingesetzt werden, beispielsweise solche Anionen, die als Kombination einer Lewis-Säure, wie BF₃, B(C₆F₅)₃, AlCl₃, SnF₂, Sn(CF₃SO₃)₂, SnCl₂ oder GeCl₂, mit einer Protonsäure, vorzugsweise einer solchen mit einem pKa-Wert von weniger als 5, wie einer Sulfonsäure, wie CF₃SO₃H, CH₃SO₃H, oder einer Halogenwasserstoffsäure, wie HF, HCl, oder einer Kombination einer Lewis-Säure mit einem Alkohol. Beispiele solcher Komplex-Anionen sind BF₄⁻, SnCl₃⁻, [SnCl₂·CF₃SO₃]⁻ und PF₆⁻.

Die Carbonylierungen können vorteilhaft bei einer Temperatur im Bereich von 30 bis 200°C, vorzugsweise 50 bis 180°C durchgeführt werden. Der Druck kann in weiten Bereichen variieren. Vorteilhaft kann der Druck in einem Bereich von 1 bis 200 bar, inbesondere im Bereich von 5 bis 90 bar, liegen. Dabei kommt insbesondere ein solcher Druck in Betracht, daß die Carbonylierung vorteilhaft in flüssiger Phase stattfindet.

Kohlenmonoxid kann vorteilhaft im molaren Überschuß gegenüber "HY" eingesetzt werden.

Die Verbindung "HY" kann vorteilhaft im molaren Verhältnis gegenüber der ungesättigten Verbindung im Bereich von 10:1 bis 1:10, vorzugsweise im Bereich von 5:1 bis 1:5, insbesondere im Bereich von 2:1 bis 1:2 eingesetzt werden.

Die Menge an Katalysator ist an sich nicht kritisch und kann in einem weiten Bereich variiert werden. Im allgemeinen hat sich eine Menge im Bereich von 10⁻⁸ bis 10⁻¹, vorzugsweise 10⁻⁷ bis 10⁻² mol Atom Palladium pro mol ungesättigte Verbindung als vorteilhaft erwiesen.

Zur Herstellung des erfindungsgemäßen Systems kann die molare Menge an Komponente (II) im allgemeinen etwas höher sein als die molare Menge an Palladium oder Palladium-Kation.

Als besonders vorteilhaft hat sich ein molares Verhältnis von Ligand zu Palladium im Bereich von 0,5 bis 10 erwiesen.

Im Falle von besonders aktiven Systemen kommt ein äquimolarer Einsatz von Diphosphin und Palladium in Betracht.

Vorteilhaft kann das molare Verhältnis von Ligand zu Palladium im Bereich von 1 bis 3, vorzugsweise 1 bis 2, liegen.

Im Falle der Gegenwart von Sauerstoff kann eine geringfügig höhere Menge vorteilhaft sein.

Die Menge an Anionenquelle kann vorteilhaft im Bereich von 0,5 bis 200, vorzugsweise 1 bis 80 mol, pro mol Palladium liegen. In dem erfindungsgemäßen Verfahren können Ausgangsmaterial und Produkt der Carbonylierungsreaktion als flüssiges Verdünnungsmittel wirken. In einem solchen Fall kann der Einsatz eines weiteren flüssigen Verdünnungsmittel entbehrlich sein. Vorteilhaft kommt der Einsatz eines weiteren flüssigen Verdünnungsmittels in Betracht, wie eines Kohlenwasserstoffs, beispielsweise eines Alkans, wie eines verzweigten oder unverzweigten Alkans, oder eines Ethers, wie 2,5,8-Trioxanonan (Diglyme), Diethylether, Diphenylether oder Anisol, Sulfons, wie Sulfolan, oder eines aromatischen Kohlenwagserstoffs, wie Toluol.

Geeignete Koreaktanten bei der Carbonylierung umfassen Verbindungen enhaltend ein nukleophiles Zentrum und ein bewegliches Wasserstoffatom.

Als bevorzugte nukleophile Verbindungen kommen molekularer Wasserstoff, Wasser, Alkohole, wie Monoalkohole, beispielsweise Methanol, Ethanol, i-Propanol, n-Propanol, 1-n-Butanol, s-Butanol, t-Butanol, i-Butanol, wie Polyalkohole, beispielsweise Ethylenglykol, 1,2-Propandiol, 1,3-Propandiol, 1,4-Butandiol, Glyzerin, Thiole, primäre und sekundäre Amine, Polyamine, Amide und Polyamide, wie Diethylamin, N,N-Dimethylethylendiamin, aromatische Alkohole, "Carbonsäure, wie Essigsäure, Pivalinsäure und Propionsäure, insbesondere molekularer Wasserstoff, Monoalkohole mit 1 bis 6 Kohlenstoffatomen, Dialkohole mit 2 bis 6 Kohelnstoffatomen, besonders bevorzugt Monoalkohole mit 1 bis 6 Kohlenstoffatomen, wie Methanol, Ethanol, i-Propanol, n-Propanol, 1-n-Butanol, s-Butanol, t-Butanol, i-Butanol, in Betracht.

Der Einsatz solcher Monoalkohole ermöglicht die Herstellung von wertvollen Zwischenprodukten, wie cis-2-Pentensäuremethylester, trans-2-Pentensäuremethylester, cis-3-Pentensäuremethylester, trans-3-Pentensäuremethylester, 4-Pentensäuremethylester. Diese Zwischenprodukte können beispielsweise wie bereits eingangs erwähnt bei der Herstellung von Polymeren, insbesondere Polyamid 6 und Polyamid 66, eingesetzt werden.

Eine andere Gruppe bevorzugter Koreaktanten umfaßt die Alkylphenole, wobei eine oder mehrere der mit dem Phenol-Teil verknüpften Alkyl-Gruppen vorzugsweise bis zu 30, bevorzugt 6 bis 22, Kohlenstoffatome enthält. Die beschriebene Carbonylierung führt in diesem Fall zu Alkylphenylestern, die Anwendung finden können als synthetische Schmiermittel, beispielweise in industriellen Anwendungen oder im Automobil-Bereich.

Die Erfindung wird anhand der folgenden; nicht einschränkenden Beispiele näher erläutert.

### Beispiele

### Beispiel 1

In einen 270 ml Autoklave mit Magnetrührer wurden 107 g Diphenylether, 20 ml Methanol, 20 ml Butadien, 0.5 mmol Palladiumacetat, 1 mmol 1,4-P,P'-di(2-phospha-1,3,5,7-tetramethyl-6,9,10-trioxatricyclo[3.3.1.1{3,7}]decyl)-butan und 3 0 mmol 2, 4, 6-Trimethylbenzoesäure unter Stickstoff-Atmosphäre eingefüllt. Der Autoklav wurde verschlossen und nach Aufpressen von 40 bar Kohlenmonoxid auf 150 °C erwärmt. Nach Erreichen dieser-Temperatur wurde der Druck durch Nachpressen von Kohlenmonoxid 10 Stunden lang konstant gehalten. Nach der Abkühlung und der Entspannung des Autokläven wurde die Reaktionslösung herausgezogen. Es haridelt sich um eine klare gelbe Lösung ohne Palladium-Niederschlag. Der Umsatz und die Selektivität zu den verschiedenen Produkten werden durch eine GC-Analyse der Lösung gemessene.

Der Umsatz an Butadien betrug 82 %, die Selektivität zu Pentensäuremethylester 80.5 %.

### Vergleichsbeispiel A

Beispiel 1 wurde mit 1 mmol 1,3-P,P'-di(2-phospha-1,3,5,7-tetramethyl-6,9,10-trioxatricyclo [3.3.1.1{3,7}]decyl)-propan anstatt 1 mmol 1,4-P,P'-di (2-phospha-1,3,5,7-tetramethyl-6,9,10-trioxatricyclo[3.3.1.1{3,7}]decyl)-butan wiederholt. Die Reaktionslösung war klar gelb ohne Palladium-Nierderschlag.

Der Umsatz an Butadien betrug 81 %, die Selektivität zu Pentensäuremethylester 22.8 %.

### Vergleichsbeispiel B

Beispiel 1 wurde mit 1 mmol 1,4-Bis(diphenylphosphino)-butan anstatt 1 mmol 1,4-P,P'-di(2-phospha-1,3,5,7-tetramethyl-6,9,10-trioxatricyclo[3.3.1.1{3,7}]decyl)-butan wiederholt. Nach der Reaktion enthielt das Reaktionsgemisch einen schwarzen Palladium-Niederschlag. Der Umsatz an Butadien betrug 97 %, die Selektivität zu Pentensäuremethylester 68.8 %.

### Vergleichsbeispiel C

Beispiel 1 wurde mit 1 mmol 1,3-Bis(dicyclohexylphosphino)-ethan anstatt 1 mmol 1,4-P,P'-di(2-phospha-1,3,5,7-tetramethyl-6,9,10-trioxatricyclo[3.3.1.1{3,7}]decyl)-butan und mit 5 mmol 2,4,6-Trimethylbenzoesäure anstatt 30 mmol wiederholt. Die Reaktionslösung war nach der Reaktion klar gelb ohne Palladium-Nierderschlag.

Der Umsatz an Butadien) betrug 55 %, die Selektivität zu Pentensäuremethylester 8.9 %.

### Vergleichsbeispiel D

Beispiel 1 wurde mit 1 mmol 1,3-Bis(1,5-cyclooctylenphosphino)propan anstatt 1 mmol 1,4-P,P'-di(2-phospha-1,3,5,7-tetramethyl-6,9,10-trioxatricyclo[3.3.1.1{3,7}]decyl)-butan wiederholt. Nach der Reaktion enthielt das Reaktionsgemisch einen schwarzen Palladium-Niederschlag.

Der Umsatz an Butadien betrug 73 %, die Selektivität zu Pentensäuremethylester 64.5 %.

## Patentansprüche

1. Diphosphin der Formel
R¹>P-(CH₂)ₙ-PR²R³
wobei
R¹ ein bivalentes Radikal ist, das zusammen mit dem Phosphor-Atom, mit dem es verknüpft ist, eine unsubstituierte oder substituierte 2-Phospha-tricyclo[3.3.1.1{3,7}]decyl-Gruppe oder ein Derivat hiervon darstellt, in dem eines oder mehrere der Kohlenstoffatome durch Heteroatome ersetzt sind,
R², R³ unabhängig voneinander ein einwertiges Radikal mit 1 bis 20 Atomen oder ein gemeinsames zweiwertiges Radikal mit 2 bis 20 Atomen,
n 4 oder 5 sind,
und deren Gemische.

2. Diphosphin nach Anspruch 1, wobei R¹ in einer oder mehrerender Positionen 1, 3, 5 oder 7 durch ein einwertiges Radikal R⁴ mit 1 bis 20 Atomen substituiert ist.

3. Diphosphin nach Anspruch 2, wobei R⁴ ein Radikal ausgewählt aus der Gruppe bestehend aus Methyl, Trifluormethyl, Ethoxy, Phenyl und 4-Dodecylphenyl ist.

4. Diphosphin nach den Ansprüchen 1 bis 3, wobei R¹ in jeder der Positionen 1, 3, 5, und 7 substituiert ist durch, vorzugsweise gleiche, Radikale R⁴.

5. Diphosphin nach den Ansprüchen 1 bis 4, wobei R¹ Sauerstoff oder Schwefel im Gerüst trägt, vorzugsweise in der Position 6, 9 und 10.

6. Diphosphin nach den Ansprüchen 1 bis 5, wobei R¹ eine. 2-Phospha-1,3,5,7-tetramethyl-6,9,10-trioxoadamantyl-Gruppe ist.

7. Diphosphin nach den Ansprüchen 1 bis 6, wobei R² und R³ zusammen eine Gruppe R¹ gemäß den Ansprüchen 1 bis 6, vorzugsweise eine mit R¹ identische Gruppe, darstellen.

8. Diphosphin nach den Ansprüchen 1 bis 7 mit n gleich 4.

9. Diphosphin nach den Ansprüchen 1 bis 7 mit n gleich 5.

10. Diphosphin nach Anspruch 1 ausgewählt aus der Gruppe bestehend aus 1,4-P,P'-di(2-phospha-1,3,5,7-tetramethyl-6,9,10-trioxatricyclo[3.3.1.1{3,7}]decyl)-butan, 1,4-P,P'-di-perfluoro-(2-phospha-1,3,5,7-tetramethyl-6,9,10-trioxatricyclo[3.3.1.1{3,7}]decyl)-butan, 1,4-P,P'-di(2-phospha-1,3,5,7-tetra(trifluormethyl)-6,9,10-trioxatricyclo[3.3.1.1{3,7}]decyl)-butan, vorzugsweise 1,4-P,P'-di(2-phospha-1,3,5,7-tetramethyl-6,9,10-trioxatricyclo[3.3.1.1{3,7}]decyl)-butan.

11. Diphosphin nach Anspruch 1 ausgewählt aus der Gruppe bestehend aus 1,5-P,P'-di(2-phospha-1,3,5,7-tetramethyl-6,9,10-trioxatricyclo[3.3.1.1{3,7}]decyl)-pentan, 1,5-P,P'-di-perfluoro-(2-phospha-1,3,5,7-tetramethyl-6,9,10-trioxatricyclo[3.3.1.1{3,7}]decyl)-pentan, 1,5-P,P'-di(2-phospha-1,3,5,7-tetra(trifluormethyl)-6,9,10-trioxatricyclo[3.3.1.1{3,7}]decyl)-pentan, vorzugsweise 1,5-P,P'-di(2-phospha-1,3,5,7-tetramethyl-6,9,10-trioxatricyclo[3.3.1.1{3,7}]decyl)-pentan.

12. Als Carbonylierungskatalysator geeignetes System enthaltend
(I) Palladium oder eine Palladiumverbindung und
(II) ein Diphosphin gemäß den Ansprüchen 1 bis 11.

13. Verfahren zur Herstellung einer Alkencarbonsäure oder eines ihrer Derivate durch Carbonylierung eines konjugierten Diens in Anwesenheit einer hydroxylgruppenhaltigen Verbindung in flüssiger Phase, **dadurch gekennzeichnet, daß** man die Carbonylierung in Gegenwart eines als Carbonylierungskatalysator geeigneten Systems aus
(I) Palladium oder einer Palladiumverbindung
(II) einem Diphosphin gemäß den Ansprüchen 1 bis 11 durchführt.

14. Verfahren nach Anspruch 13, wobei man als konjugiertes Dien 1,3-Butadien einsetzt unter Erhalt von Pentensäure oder eines ihrer Derivate, vorzugsweise von Pentensäureester.

## Claims

1. A diphosphine of the formula
R¹>P-(CH₂)ₙ-PR²R³
where
R¹ is a divalent radical which together with the phosphorus atom to which it is linked forms an unsubstituted or substituted 2-phosphatricyclo[3.3.1.1{3,7}]decyl group or a derivative thereof in which one or more of the carbon atoms are replaced by heteroatoms,
R², R³ are each, independently of one another, a monovalent radical having from 1 to 20 atoms or together form a divalent radical having from 2 to 20 atoms,
n is 4 or 5,
or a mixture of such diphosphines.

2. The diphosphine according to claim 1, wherein R¹ is substituted in one or more of the positions 1, 3, 5 and 7 by a monovalent radical R⁴ having from 1 to 20 atoms.

3. The diphosphine according to claim 2, wherein R⁴ is a radical selected from the group consisting of methyl, trifluoromethyl, ethoxy, phenyl and 4-dodecylphenyl.

4. The diphosphine according to any of claims 1 to 3, wherein R¹ is substituted in each of the positions 1, 3, 5, and 7 by preferably identical radicals R⁴.

5. The diphosphine according to any of claims 1 to 4, wherein R¹ contains oxygen or sulfur in the skeleton, preferably in the positions 6, 9 and 10.

6. The diphosphine according to any of claims 1 to 5, wherein R¹ is a 2-phospha-1,3,5,7-tetramethyl-6,9,10-trioxoadamantyl group.

7. The diphosphine according to any of claims 1 to 6, wherein R² and R³ together form a group R¹ as set forth in any of claims 1 to 6, preferably a group identical to R¹.

8. The diphosphine according to any of claims 1 to 7 in which n is 4.

9. The diphosphine according to any of claims 1 to 7 in which n is 5.

10. The diphosphine according to claim 1 selected from the group consisting of 1,4-P,P'-di(2-phospha-1,3,5,7-tetramethyl-6,9,10-trioxatricyclo[3.3.1.1{3,71}]decyl)butane. 1,4-P,P'-diperfluoro(2-phospha-1,3,5,7-tetramethyl-6,9,10-trioxatricyclo[3.3.1.1{3,7}]decyl)butane, 1,4-P,P'-di(2-phospha-1,3,5,7-tetra(trifluoromethyl)-6,9,10-trioxatricyclo[3.3.1.1{3,7}]decyl)butane, preferably 1,4-P,P'-di(2-phospha-1,3,5,7-tetramethyl-6,9,10-trioxatri-cyclo[3.3.1.1{3,7}]decyl)butane.

11. The diphosphine according to claim 1 selected from the group consisting of 1,5-P,P'-di(2-phospha-1,3,5,7-tetramethyl-6,9,10-trioxatricyclo[3.3.1.1{3,7}]decyl)pentane, 1,5-P,P'-diperfluoro(2-phospha-1,3,5,7-tetramethyl-6,9,10-trioxatricyclo[3.3.1.1{3,7}]decyl]pentane, 1,5-P,P'-di(2-phospha-1,3,5,7-tetra(trifluoromethyl)-6,9,10-trioxatricyclo[3.3.1.1{3,7}]decyl)pentane, preferably 1,5-P,P'-di(2-phospha-1,3,5,7-tetramethyl-6,9,10-trioxatricyclo[3.3.1.1{3,7}]decyl)pentane.

12. A system which is suitable as carbonylation catalyst and comprises
(I) palladium or a palladium compound and
(II) a diphosphine according to any of claims 1 to 11.

13. A process for preparing an alkenecarboxylic acid or a derivative thereof by carbonylation of a conjugated diene in the presence of a hydroxyl-containing compound in the liquid phase, wherein the carbonylation is carried out in the presence of a system which is suitable as carbonylation catalyst and comprises
(I) palladium or a palladium compound and
(II) a diphosphine according to any of claims 1 to 11.

14. A process according to claim 13, wherein 1,3-butadiene is used as conjugated diene to give pentenoic acid or a derivative thereof, preferably a pentenoic ester.

## Revendications

1. Diphosphines de la formule :
R¹>P-(CH₂)ₙ-PR²R³
dans laquelle :
R¹ est un radical bivalent qui, conjointement à l'atome de phosphore auquel il est lié, représente un groupe 2-phospha-tricyclo[3.3.1.1{3,7}]décyle substitué ou non substitué ou un dérivé de celui-ci, dans lequel un ou plusieurs des atomes de carbone sont remplacés par des hétéroatomes,
R² R³ représentent, indépendamment l'un de l'autre, un radical monovalent comportant 1 à 20 atomes ou un radical bivalent commun comportant 2 à 20 atomes,
n vaut 4 ou 5,
et leurs mélanges.

2. Diphosphine suivant la revendication 1, dans laquelle R¹ est, dans une ou plusieurs des positions 1, 3, 5 ou 7, substitué par un radical monovalent R⁴ comportant 1 à 20 atomes.

3. Diphosphine suivant la revendication 2, dans laquelle R⁴ est un radical choisi parmi le groupe constitué de méthyle, de trifluorométhyle, d'éthoxy, de phényle et de 4-dodécylphényle.

4. Diphosphine suivant les revendications 1 à 3, dans laquelle R¹ est, dans chacune des positions 1, 3, 5 et 7, substitué par des radicaux R⁴, de préférence identiques.

5. Diphosphine suivant les revendications 1 à 4, dans laquelle R¹ porte de l'oxygène ou du soufre dans le squelette, de préférence dans la position 6, 9 et 10.

6. Diphosphine suivant les revendications 1 à 5, dans laquelle R¹ est un groupe 2-phospha-1,3,5,7-tétraméthyl-6,9,10-trioxoadamantyle.

7. Diphosphine suivant les revendications 1 à 6, dans laquelle R² et R³ représentent conjointement un groupe R¹ suivant les revendications 1 à 6, de préférence un groupe identique à R¹.

8. Diphosphine suivant les revendications 1 à 7, où n est égal à 4.

9. Diphosphine suivant les revendications 1 à 7, où n est égal à 5.

10. Diphosphine suivant la revendication 1, choisie parmi le groupe constitué de 1,4-P,P'-di(2-phospha-1,3,5,7-tétraméthyl-6,9,10-trioxatricyclo[3.3.1.1{3,7}décyl)-butane, de 1,4-P,P'-di-perfluoro-(2-phospha-1,3,5,7-tétraméthyl-6,9,10-trioxatricyclo[3.3.1.1{3,7}]décyl)-butane, de 1,4-P,P'-di(2-phospha-1,3,5,7-tétra(trifluorométhyl)-6,9,10-trioxatricyclo[3.3.1.1{3,7}]décyl)-butane, de préférence de 1,4-P,P'-di(2-phospha-1,3,5,7-tétraméthyl-6,9,10-trioxatricyclo[3.3.1.1{3,7}]décyl)-butane.

11. Diphosphine suivant la revendication 1, choisie parmi le groupe constitué de 1,5-P,P'-di(2-phospha-1,3,5,7-tétraméthyl-6,9,10-trioxatricyclo[3.3.1.1{3,7}]décyl)-pentane, de 1,5-P,P'-di-perfluoro-(2-phospha-1,3,5,7-tétraméthyl-6,9,10-trioxatricyclo[3.3.1.1{3,7}]décyl)-pentane, de 1,5-P,P'-di(2-phospha-1,3,5,7-tétra(trifluorométhyl)-6,9,10-trioxatricyclo[3.3.1.1{3,7}]décyl)-pentane, de préférence de 1,5-P,P'-di(2-phospha-1,3,5,7-tétraméthyl-6,9,10-trioxatricyclo[3.3.1.1{3,7}]décyl)-pentane.

12. Système approprié comme catalyseur de carbonylation, contenant
(I) du palladium ou un composé de palladium, et
(II) une diphosphine suivant les revendications 1 à 11.

13. Procédé de préparation d'un acide alcènecarboxylique ou d'un de ses dérivés par carbonylation d'un diène conjugué en présence d'un composé contenant des groupes hydroxyle en phase liquide, **caractérisé en ce qu'**on effectue la carbonylation en présence d'un système approprié comme catalyseur de carbonylation à base
(I) de palladium ou d'un composé de palladium,
(II) d'une diphosphine suivant les revendications 1 à 11.

14. Procédé suivant la revendication 13, dans lequel, comme diène conjugué, on met en oeuvre du 1,3-butadiène avec obtention d'acide penténique ou d'un de ses dérivés, de préférence d'ester d'acide penténique.
